# EUROPEAN PATENT SPECIFICATION

(11) **EP 4 382 045 B1**
(45) Date of publication and mention of the grant of the patent: **03.09.2025**
(21) Application number: 22852180.3
(22) Date of filing: 02.08.2022
(51) Int. Cl.: A61B 5/20, A61B 5/22, A61B 5/391, A61B 5/00

(54) **NEUROELECTROPHYSIOLOGICAL MONITORING DEVICE**
VORRICHTUNG ZUR NEUROELEKTROPHYSIOLOGISCHEN ÜBERWACHUNG
DISPOSITIF DE SURVEILLANCE NEUROÉLECTROPHYSIOLOGIQUE

(30) Priority: 03.08.2021 CN 202110886485; 03.08.2021 CN 202110887745; 03.08.2021 CN 202110886504
(43) Date of publication of application: 12.06.2024
(73) Proprietor: BEIJING HUASHENSHENGDIAN MEDTECH CO LTD., Changping District Beijing 100085 (CN)
(72) Inventor: LIU, Xianzeng, Beijing 100085 (CN); LIU, Jingyi, Beijing 100085 (CN)
(74) Representative: Sun, Yiming
(86) International application number: PCT/CN2022/109694
(87) International publication number: WO 2023/011468

(56) References cited:
- CN-A- 102 573 617
- CN-A- 102 711 647
- CN-A- 106 073 807
- CN-U- 208 799 249
- CN-U- 215 384 035
- CN-U- 215 384 093
- CN-U- 215 384 094
- US-A- 4 063 548
- US-A- 4 063 548
- US-A1- 2007 010 761
- US-A1- 2009 222 058
- US-A1- 2012 265 044
- US-A1- 2020 268 302
- AMARENCO G ET AL: "COUGH ANAL REFLEX: STRICT RELATIONSHIP BETWEEN INTRAVESICAL PRESSURE AND PELVIC FLOOR MUSCLE ELECTROMYOGRAPHIC ACTIVITY DURING COUGH. URODYNAMIC AND ELECTROPHYSIOLOGICAL STUDY", JOURNAL OF UROLOGY, LIPPINCOTT WILLIAMS & WILKINS, BALTIMORE, MD, US, vol. 173, no. 1, 1 January 2005 (2005-01-01), pages 149 - 152, XP027854694, ISSN: 0022-5347, [retrieved on 20050101]

## Description

### FIELD

The present invention relates to medical instruments, and more particularly relates to a neuroelectrophysiological monitoring apparatus.

### BACKGROUND

At present, intraoperatively monitored signals in relevant techniques include an intravesical pressure, an intrarectal pressure, an external urethra sphincter electromyographic activity, and an external anal sphincter electromyographic activity. However, a conventional monitoring apparatus is designed to only monitor one certain signal during a surgical procedure due to its structural limitation, incapable of monitoring multiple signals simultaneously. Detection of the intravesical pressure, the intrarectal pressure, myoelectric recordings of the external urethra sphincter, and myoelectric recordings of the external anal sphincter relies on multiple sets of invasive monitoring instruments that work separately, resulting in a large trauma as well as a high expense to a patient; in addition, conventional measurement approaches are incapable of simultaneously measuring the intravesical pressure, the intrarectal pressure, myoelectric recordings of the external urethra sphincter, and myoelectric recordings of the external anal sphincter, which leads to deteriorated operative safety and increased postoperative risks. US 2020/268302 A1 discloses systems and methods for providing computer-aided diagnosis of lower urinary tract dysfunction/disorders based on data obtained from the urodynamic test. US 4 063 548 A discloses a method and an apparatus for analyzing micturition disturbances. US 2009/222058 A1 discloses a wearable neuromodulation device configured for insertion into a pelvic orifice of the human body for treating urinary incontinence, faecal incontinence, muscle wastage, spasm and/or spasticity. US 2021/265044 A1 discloses a system and a method for client-side compression and extraction of medical imaging data. AMARENCO G ET AL: "COUGH ANAL REFLEX: STRICT RELATIONSHIP BETWEEN INTRAVESTICAL PRESSURE AND PELVIC FLOOR MUSCLE ELECTROMYOGRAPHIC ACTIVITY DURING COUGH. URODYNAMIC AND ELECTROPHYSIOLOGICAL STUDY", JOURNAL OF UROLOGY, LIPPINCOTT WILLIAMS & WILKINS, BALTIMORE, MD, US, vol. 173, no. 1, 1 January 2005 (2005-01-01), pages 149-152, XP027854694, ISSN: 0022-5347 discloses that pelvic floor muscle contraction increases with importance of intra-abdominal pressure generated during stress.

### SUMMARY

The disclosure intends to at least solve one of the above and other technical problems in conventional technologies. To this end, an object of the disclosure is to provide a neuroelectrophysiological monitoring apparatus, which may simultaneously measure an intravesical pressure, an intrarectal pressure, myoelectric recordings of the external urethra sphincter, and myoelectric recordings of the external anal sphincter, thereby eliminating a need of deploying multiple sets of monitoring instruments; as such, integrity of the neuroelectrophysiological monitoring apparatus is improved, operative safety is enhanced, and postoperative risks are reduced.

The present invention is defined by the independent claim. Advantageous embodiments are described in the dependent claims, the following description and the drawings. A neuroelectrophysiological monitoring apparatus according to the disclosure comprises: a urethra testing device, the urethra testing device being provided with a bladder pressure measuring member and a first myoelectricity measuring member, the bladder pressure measuring member being set in a patient's bladder and adapted to measure the patient's intravesical pressure, the first myoelectricity measuring member being adapted to contact an external urethra sphincter to measure an external urethra sphincter electromyographic activity; a rectum testing device, the rectum testing device being provided with a rectal pressure measuring member and a second myoelectricity measuring member, the rectal pressure measuring member being set in the patient's rectum and adapted to measure the patient's intrarectal pressure, the second myoelectricity measuring member being adapted to contact an external anal sphincter to measure an external anal sphincter electromyographic activity; and a monitoring module, the monitoring module being electrically connected to the bladder pressure measuring member, the first myoelectricity measuring member, the rectal pressure measuring member, and the second myoelectricity measuring member, respectively.

**In** brief, provided with the urethra testing device configured to measure a patient's intravesical pressure and external urethra sphincter electromyographic activity, the rectum testing device configured to measure the patient's intrarectal pressure and external anal sphincter electromyographic activity, and the monitoring module configured to monitor the intravesical pressure, the intrarectal pressure, the external urethra sphincter electromyographic activity, and the external anal sphincter electromyographic activity, the neuroelectrophysiological monitoring apparatus according to the disclosure is enabled to simultaneously monitor the intravesical pressure, the intrarectal pressure, the external urethra sphincter electromyographic activity, and the external anal sphincter electromyographic activity, eliminating a need of deploying multiple sets of monitoring instruments, thereby improving integrity of the neuroelectrophysiological monitoring apparatus as well as operative safety, with reduced postoperative risks.

According to one embodiment of the disclosure, the bladder pressure measuring member comprises: a first communication tube, the first communication tube being adapted to deliver a medium, one end of the first communication tube being open and set in the patient's bladder, opposite end of the first communication tube extending outside the patient's body; a first pressure transducing device, the first pressure transducing device having a first testing end and a first signal output end, the first testing end communicating with the first communication tube and being adapted to measure a pressure of the medium in the first communication tube, the first signal output end being in communicative connection to the monitoring module, the first pressure transducing device being adapted to convert the pressure of the medium in the first communication tube to an intravesical pressure signal and to transmit the intravesical pressure signal to the monitoring module via the first signal output end.

According to one embodiment of the disclosure, the opposite end of the first communication tube is provided with a medium injecting device, the medium injecting device selectively injecting the medium into the first communication tube.

According to one embodiment of the disclosure, the first myoelectricity measuring member is disposed at an outer periphery of the first communication tube.

According to one embodiment of the disclosure, the first myoelectricity measuring member is formed of a plurality of first electromyography electrodes, the plurality of first electromyography electrodes being arranged at intervals in an extending direction of the first communication tube or in a circumferential direction of the first communication tube.

According to one embodiment of the disclosure, the neuroelectrophysiological monitoring apparatus further comprises: a first communication tube anchoring device, the first communication tube anchoring device being disposed at one end of the first communication tube, the first communication tube anchoring device being selectively deformed to be tensioned in the patient's bladder, an expandable cavity being formed inside the first communication tube anchoring device, the expandable cavity communicating with a syringe connection tube so that the medium is injected into the expandable cavity to control deformation of the communication tube anchoring device.

According to one embodiment of the disclosure, the rectal pressure measuring member comprises: a second communication tube, the second communication tube being adapted to deliver the medium, one end of the second communication tube being set in the patient's rectum, opposite end of the second communication tube extending outside the patient's body; an intrarectal expandable balloon, the intrarectal expandable balloon being disposed at one end of the second communication tube and communicating with the second communication tube, an outer peripheral wall of the intrarectal expandable balloon abutting against an inside lining of the patient's rectum; and a second pressure transducing device, the second pressure transducing device having a second testing end and a second signal output end, the second testing end communicating with the second communication tube and being adapted to measure a pressure of the medium in the second communication tube, the second signal output end being in communicative connection to the monitoring module, the second pressure transducing device being adapted to convert the pressure of the medium to an intrarectal pressure signal and transmit the intrarectal pressure signal to the monitoring module via the second signal output end.

According to one embodiment of the disclosure, the opposite end of the second communication tube is adaptable to be connected to the medium injecting device, the medium injecting device selectively injecting the medium into the second communication tube.

According to one embodiment of the disclosure, the neuroelectrophysiological monitoring apparatus further comprises: a three-way tube, the three-way tube having a first interface, a second interface, and a third interface which selectively communicate with each other, the first interface being connected to the opposite end of the second communication tube, the second interface being connected to the second testing end of the second pressure transducing device, the third interface being connected to the medium injecting device.

According to one embodiment of the disclosure, the second myoelectricity measuring member is disposed at an outer periphery of the second communication tube.

According to one embodiment of the disclosure, the second myoelectricity measuring member is formed of a plurality of second electromyography electrodes, the plurality of second electromyography electrodes being arranged at intervals in an extending direction of the second communication tube or in a circumferential direction of the first communication tube.

According to one embodiment of the disclosure, a plurality of scale markers arranged at intervals in the extending direction of the second communication tube are provided on an outer peripheral wall of the second communication tube.

Additional aspects and advantages of the disclosure will be partially given in the detailed description below, or partially become apparent through the detailed description below, or be understood through implementing the disclosure.

### BRIEF DESCRIPTION OF THE DRAWINGS

Fig. 1 is a structural schematic diagram of a neuroelectrophysiological monitoring apparatus according to a first aspect of embodiments of the disclosure not falling under the scope of protection;
Fig. 2 is a structural schematic diagram of a neuroelectrophysiological monitoring apparatus according to a second aspect of embodiments of the disclosure not falling under the scope of protection;
Fig. 3 is a structural schematic diagram of a neuroelectrophysiological monitoring apparatus according to present invention;
Fig. 4 is a structural schematic diagram of a ring-shaped electromyography electrode according to some embodiments of the disclosure not falling under the scope of protection;
Fig. 5 is a sectional view of a strip-shaped electromyography electrode according to some embodiments of the disclosure not falling under the scope of protection.

Reference Numerals:
neuroelectrophysiological monitoring apparatus 100;
communication tube 111, interconnecting catheter 112, testing end 113, medium injecting device 114, electromyography electrode 121, electromyography electrode connecting harness 122, monitoring module 13, communication tube anchoring device 14, syringe connection tube 15, urethra catheter 161, urine collection bag 162, first regulating valve 17, second regulating valve 18, intravesical fluid outlet 19;
neuroelectrophysiological monitoring apparatus 200;
communication tube 211, interconnecting catheter 212, testing end 213, medium injecting device 214, intrarectal expandable balloon 215, electromyography electrode 221, electromyography electrode connecting harness 222, monitoring module 23, third regulating valve 24;
neuroelectrophysiological monitoring apparatus 300;
first communication tube 3111, first interconnecting catheter 3112, first testing end 3113, first electromyography electrode 3121, first electromyography electrode connecting harness 3122, communication tube anchoring device 313, syringe connection tube 314, urethra catheter 3151, urine collection bag 3152, fourth regulating valve 316, fifth regulating valve 317, intravesical fluid outlet 318;
second communication tube 3211, second interconnecting catheter 3212, second testing end 3213, intrarectal expandable balloon 3214, second electromyography electrode 322, second electromyography electrode connecting harness 323, sixth regulating valve 324;
monitoring module 33, first medium injecting device 34, second medium injecting device 35;
ring-shaped electromyography electrode 1211, strip-shaped electromyography electrode 1212.

### DETAILED DESCRIPTION

Hereinafter, embodiments of the disclosure will be described in detail. Examples of the embodiments are illustrated in the drawings. Throughout the specification, identical or like reference numerals represent same or similar elements or elements with same or similar functions. The embodiments described with reference to the drawings are exemplary, only intended for explaining the disclosure, which shall not be understood as limitations to the disclosure.

A neuroelectrophysiological monitoring apparatus according to a first aspect of embodiments of the disclosure will be described *infra,* the neuroelectrophysiological monitoring apparatus being configured to measure a patient's intravesical pressure and external urethra sphincter electromyographic activity.

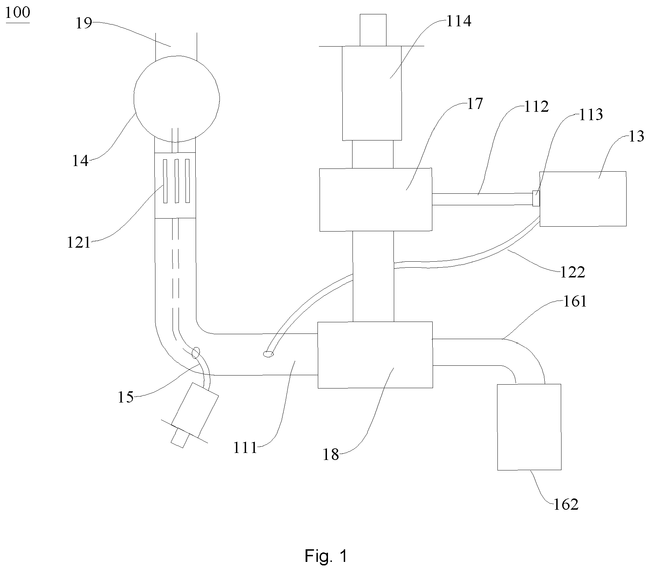

Hereinafter, a neuroelectrophysiological monitoring apparatus 100 according to the disclosure not falling under the scope of protection will be described with reference to Fig. 1. The neuroelectrophysiological monitoring apparatus 100 comprises a bladder pressure measuring member, a myoelectricity measuring member, and a monitoring module 13. The bladder pressure measuring member is set in a patient's bladder and adapted to measure an intravesical pressure of the patient, the myoelectricity measuring member is disposed adjacent to the bladder pressure measuring member and adapted to contact the external urethra sphincter to measure an external urethra sphincter electromyographic activity, and the monitoring module 13 is connected to the bladder pressure measuring member and the myoelectricity measuring member, respectively, to monitor the patient's intravesical pressure and external urethra sphincter electromyographic activity.

At present, intraoperatively monitored signals in relevant techniques include an intravesical pressure and an external urethra sphincter electromyographic activity. However, a conventional monitoring instrument is designed to only monitor one certain signal during a surgical procedure due to its structural limitation, incapable of monitoring multiple signals simultaneously. Detection of the intravesical pressure and electromyography recordings of the external urethra sphincter relies on multiple sets of invasive monitoring instruments that work separately, resulting in a large trauma as well as a high expense to the patient; in addition, conventional measurement approaches are incapable of simultaneously measuring the intravesical pressure and the electromyography recordings of external urethra sphincter, which leads to deteriorated operative safety and increased postoperative risks.

Specifically, the neuroelectrophysiological monitoring apparatus 100 comprises a bladder pressure measuring member, a myoelectricity measuring member, and a monitoring module 13. The bladder pressure measuring member is disposed in a patient's bladder and adapted to measure the patient's intravesical pressure; the myoelectricity measuring member is disposed adjacent to the bladder pressure measuring member and adapted to contact the external urethra sphincter to measure an external urethra sphincter electromyographic activity via the contact between the myoelectricity measuring member and the external urethra sphincter; the monitoring module 13 is connected to the bladder pressure measuring member and the myoelectricity measuring member, respectively, so that the bladder pressure measuring member and the myoelectricity measuring member may transmit signals to the monitoring module 13, so that the monitoring module 13 monitors the intravesical pressure and the external urethra sphincter electromyographic activity of the patient based on the signals received.

In brief, with the pressure measuring member configured to measure a patient's intravesical pressure, the myoelectricity measuring member configured to measure the external urethra sphincter electromyographic activity, and the monitoring module 13 configured to monitor the intravesical pressure and the external urethra sphincter electromyographic activity, the neuroelectrophysiological monitoring apparatus 100 according to the disclosure is enabled to simultaneously monitor the intravesical pressure and the external urethra sphincter electromyographic activity, eliminating a need to deploy multiple monitoring instruments, which improves operative safety and reduces postoperative risks.

According to one embodiment of the disclosure, the bladder pressure measuring member comprises a communication tube 111 and a pressure transducing device, the communication tube 111 being adapted to deliver a medium, wherein the medium may be a liquid medicine such as physiological saline. One end of the communication tube 111 is open and set in the patient's bladder, and the opposite end of the communication tube 111 extends out of the patient's body. The communication tube 111 is adapted to deliver the medium, whereby the medium is delivered from the external to the patient's body, or delivered from the inside the patient' s body to the external.

The pressure transducing device has a testing end 113 and a signal output end, the testing end 113 being in communication with the communication tube 111 and configured to measure a pressure of the medium in the communication tube 111, wherein an interconnecting catheter 112 is provided between the testing end 113 and the communication tube 111, one end of the interconnecting catheter 112 being connected to the communication tube 111, the opposite end of the interconnecting catheter 112 being connected to the testing end 113, so that the testing end 113 communicates with an internal cavity of the communication tube 111. In another embodiment of the disclosure, the interconnecting catheter 112 and the communication tube 111 may be unitarily formed. An on/off valve may be provided between the interconnecting catheter 112 and the communication tube 111, the on/off valve allowing for selective communication between the interconnecting catheter 112 and the communication tube 111.

The signal output end is connected to the monitoring module 13; the pressure transducing device may convert the pressure of the medium to an intravesical pressure signal and transmit the intravesical pressure signal to the monitoring module 13 via the signal output end, so that the monitoring module 13 monitors the intravesical pressure of the patient based on the intravesical pressure signal.

According to one embodiment of the disclosure, the opposite end of the communication tube 111 is provided with a medium injecting device 114, wherein the medium injecting device may be configured as a syringe. The medium injecting device 114 may selectively inject the medium into the communicating device 111 so that the medium may be delivered into the patient's bladder via the communication tube 111. An on/off valve may be provided between the medium injecting device 114 and the communication tube 111, the on/off valve allowing for selective communication between the medium injecting device 114 and the communication tube 111.

According to one embodiment of the disclosure, the neuroelectrophysiological monitoring apparatus 100 further comprises a first regulating valve 17, wherein the first regulating valve 17 may be configured as a three-way regulating valve; the first regulating valve 17 comprises a first port, a second port, and a third port, the first port, the second port, and the third port being in one-to-one communication with the communication tube 111, the interconnecting catheter 112, and the medium injecting device 114, respectively. Provision of the three-way regulating valve may reduce port multiplexing and plugging operations during monitoring, which facilitates a manipulator to pre-connect the monitoring apparatus, further reducing the manipulation difficulty.

When the first port communicates with the third port while the second port is shut down, the communication tube 111 communicates with the medium injecting device 114, in which case the medium injecting device 114 may selectively inject the medium into the communication tube 111 so that the medium may be delivered into the patient's bladder via the communication tube 111; when the first port communicates with the second port while the third port is shut down, the communication tube 111 communicates with the interconnecting catheter 112, in which case the pressure transducing device may convert the pressure of the medium in the interconnecting catheter 112 to an intravesical pressure signal and transmit the intravesical pressure signal to the monitoring module 13 via the signal output end, so that the monitoring module 13 monitors the patient's intravesical pressure based on the intravesical pressure signal.

In another embodiment of the disclosure, the interconnecting catheter 112 or the medium injecting device 114 may be directly connected to the communication tube 111, thereby eliminating configuration of the first regulating valve 17.

According to one embodiment of the disclosure, the myoelectricity measuring member is disposed at an outer periphery of the communication tube 111. Furthermore, when the communication tube 111 enters the patient's bladder, the myoelectricity measuring member may directly contact the external urethra sphincter. Contact with the external urethra sphincter allows for the myoelectricity measuring member to measure the external urethra sphincter electromyographic activity. The myoelectricity measuring member may be integrated at the outer periphery of the communication tube 111, ensuring that the neuroelectrophysiological monitoring apparatus 100 measures the intravesical pressure and the external urethra sphincter electromyographic activity simultaneously, thereby realizing simultaneous measurement of two parameters, which improves operative safety and reduces postoperative risks.

According to one embodiment of the disclosure, the myoelectricity measuring member comprises a plurality of electromyography electrodes 121, the plurality of electromyography electrodes 121 being arranged at intervals at the outer periphery of the communication tube 111; the plurality of electromyography electrodes 121 may obtain the external urethra sphincter electromyographic activity by touching a mucous membrane on a surface of the external urethra sphincter, and by arranging the plurality of electromyography electrodes 121 at intervals at the outer periphery of the communication tube 111, measurement accuracy and comprehensiveness may be further improved, thereby enhancing operative safety.

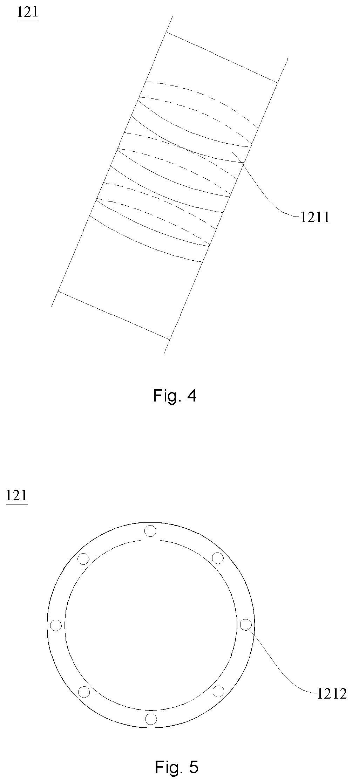

According to one embodiment of the disclosure not falling under the scope of protection, as illustrated in Fig. 5, each electromyography electrode 121 is configured to have a stripped shape, an extending direction of each electromyography electrode 121 is consistent with an extending direction of the communication tube 111, and the plurality of electromyography electrodes 121 are arranged at intervals at the outer periphery of the communication tube 111, wherein the plurality of electromyography electrodes 121 are arranged at intervals, respective electromyography electrodes 121 being insulated from each other, each electromyography electrode 121 being configured to measure an electromyography signal of the external urethra sphincter, respectively. Specifically, the number of the strip-shaped electromyography electrodes 1212 may be 2, 4, 6, 8, or more; the number of the strip-shaped electromyography electrodes 1212 may also be odd. By arranging the plurality of strip-shaped electrodes at intervals in the extending direction of the communication tube 111, at least the electromyography signals of left and right sides of the external urethra sphincter may be monitored, respectively; in addition, the electromyography signals of the external urethra sphincter corresponding to different positions in the peripheral direction of the communication tube 111 may also be monitored, thereby realizing more accurate monitoring of the electromyography activities of different positions of the external urethra sphincter.

According to one embodiment of the disclosure not falling under the scope of protection, as illustrated in Fig. 4, each electromyography electrode 121 is configured to have a ring shape and disposed surrounding the communication tube 111, the plurality of electromyography electrodes 121 being arranged at intervals in the extending direction of the communication tube 111, wherein the plurality of electromyography electrodes 121 are arranged at intervals, respective electromyography electrodes 121 being insulated from each other, each electromyography electrode 121 being configured to measure an electromyography signal of the external urethra sphincter, respectively, enabling measurement of the electromyography signals at multiple positions of the external urethra sphincter, whereby the electromyography electrodes 121 measure the electromyography signals of the external urethra sphincters more accurately. By arranging the plurality of ring-shaped electromyography electrodes 1211 at intervals surrounding the communication tube 111, it is enabled to monitor the electromyography signals of the external urethra sphincter at different depth positions corresponding to the extending direction of the communication tube 111, whereby the electromyographic activities at different positions of the external urethra sphincter may be monitored more accurately.

In a neuroelectrophysiological monitoring apparatus 100 applicable to an adult male patient, the distance between the upper end of each electromyography electrode 121 and the start of the patient's bladder neck may range from 2 cm to 5 cm, the distance being preferably 3.5 cm. In a neuroelectrophysiological monitoring apparatus 100 applicable to an adult female patient, the distance between the upper end of each electromyography electrode 121 and the start of the patient's bladder neck may range from 0.5 cm to 3 cm, the distance being preferably 1.5 cm. In a neuroelectrophysiological monitoring apparatus 100 applicable to an underage patient, the distance between the upper end of each electromyography electrode 121 and the start of the patient's bladder neck is smaller than the distance between the upper end of each electromyography electrode 121 and the start of the patient's bladder neck in the neuroelectrophysiological monitoring apparatus 100 applicable to an adult male patient or adult female patient.

According to one embodiment of the disclosure, a plurality of scale markers arranged at intervals in the extending direction of the communication tube 111 are provided on the outer peripheral wall of the communication tube 111. The scale markers are adapted to adjust the depth of the communication tube 111 inserted into the patient's urethra, ensuring that the communication tube 111 is always disposed in a safe depth, thereby improving operative safety and reducing postoperative risks.

According to one embodiment of the disclosure, the neuroelectrophysiological monitoring apparatus 100 further comprises an electromyography electrode connecting harness 122, the electromyography electrode connecting harness 122 being adapted to electrically connect the electromyography electrode 121 and the monitoring module 13, wherein the electromyography electrode connecting harness 122 is at least partially inserted into the inside or the tube wall of the communication tube 111, one end of the electromyography electrode connecting harness 122 being connected to a plurality of electromyography electrodes 121, the opposite end of the electromyography electrode connecting harness 122 being connected to the monitoring module 13, the plurality of electromyography electrodes 121 being independently and separately connectable to the monitoring module 13 via the electromyography electrode connecting harness 122, and the measured electromyography signals being transmitted to the monitoring module 13 via the electromyography electrode connecting harness 122.

According to one embodiment of the disclosure, the neuroelectrophysiological monitoring apparatus 100 further comprises a communication tube anchoring device 14, the communication tube anchoring device 14 being provided at one end of the communication tube 111, wherein the communication tube anchoring device 14 may be configured as a rubber element, and the communication tube anchoring device 14 is selectively deformed to be tensioned inside the patient's bladder. Specifically, the communication tube anchoring device 14 has a contracted state and an expanded state. When the communication tube anchoring device 14 is in the contracted state, the communication tube 111 may enter or be removed out of the patient's bladder; when the communication tube anchoring device 14 is in the expanded state, the communication tube 111 is securely disposed in the patient's bladder, uneasy to be detached.

According to one embodiment of the disclosure, an expandable cavity is formed inside the communication tube anchoring device 14, the expandable cavity being selectively expanded or contracted. The expandable cavity communicates with a syringe connecting tube 15, the syringe connecting tube 15 being adapted to inject a medium into the expandable cavity to control deformation of the communication tube anchoring device 14, wherein the medium may be a liquid or gas; the syringe connecting tube 15 injects the medium into the expandable cavity, causing expansion of the expandable cavity, so that the communication tube anchoring device 14 securely contacts the inside lining of the patient's bladder.

According to one embodiment of the disclosure, the syringe connecting tube 15 is at least partially received inside the communication tube 111 or on the tube wall of the communication tube 111, which reduces footprint of the syringe connecting tube 15 and enhances integrity of the syringe connecting tube 15.

According to one embodiment of the disclosure, the neuroelectrophysiological monitoring apparatus 100 further comprises a urethra catheter 161, one end of the urethra catheter 161 being selectively conducted to the communication tube 111, the opposite end of the urethra catheter 161 being provided with a urine collection bag 162, the urine collection bag 162 being configured to collect the residual urine or medium in the patient's bladder. Specifically, the neuroelectrophysiological monitoring apparatus 100 further comprises an intravesical fluid outlet 19, the intravesical fluid outlet 19 allowing for drainage of the residual urine or medium out of the bladder; the residual urine or medium in the bladder flows into the urethra catheter 161 via the communication tube 111 and is then delivered into the urine collection bag 162 via the urethra catheter 161 till the operation ends.

According to one embodiment of the disclosure, the neuroelectrophysiological monitoring apparatus 100 further comprises a second regulating valve 18, wherein the second regulating valve 18 may be configured as a three-way regulating valve. The second regulating valve 18 comprises a fourth port, a fifth port, and a sixth port, the fourth port, the fifth port, and the sixth port being disposed in one-to-one correspondence with a front segment of the communication tube 111, a rear segment of the communication tube 111, and the urethra catheter 161, respectively. Provision of the three-way regulating valve may reduce the port multiplexing and plugging operations during monitoring, which facilitates a manipulator to pre-connect the monitoring apparatus, further reducing manipulation difficulty.

When the fourth port and the fifth port communicate while the sixth port is shut down, the front segment of the communication tube 111 is conducted to the rear segment of the communication tube 111, in which case the medium injecting device 114 may inject a medium into the patient's bladder, facilitating monitoring of the intravesical pressure of the patient. When the fourth port and the sixth port communicate while the fifth port is shut down, the front segment of the communication tube 111 is conducted to the urethra catheter 161, in which case the residual urine or medium in the patient's bladder flows into the urethra catheter 161 along the communication tube 111 and is then delivered into the urine collection bag 162 via the urethra catheter 161 till the operation ends.

Hereinafter, a neuroelectrophysiological monitoring apparatus according to a second aspect of embodiments of the disclosure will be described, the neuroelectrophysiological monitoring apparatus being configured to measure a patient's intrarectal pressure and external anal sphincter electromyographic activity.

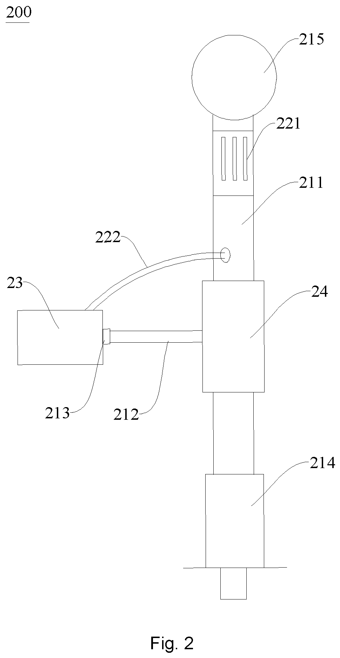

A neuroelectrophysiological monitoring apparatus 200 according to embodiments of the disclosure not falling under the scope of protection will be described below with reference to Fig. 2, the neuroelectrophysiological monitoring apparatus 200 comprising a rectal pressure measuring member, a myoelectricity measuring member, and a monitoring module 23. The rectal pressure measuring member is disposed in the patient's rectum and adapted to measure the patient's intrarectal pressure; the myoelectricity measuring member is disposed adjacent to the rectal pressure measuring member and adapted to contact with a mucous membrane on a surface of the external anal sphincter to measure an external anal sphincter electromyographic activity; and the monitoring module 23 is connected to the rectal pressure measuring member and the myoelectricity measuring member, respectively, so as to monitor the patient's intrarectal pressure and external anal sphincter electromyographic activity.

At present, intraoperatively monitored signals in relevant techniques include an intrarectal pressure and an external anal sphincter electromyographic activity. However, a conventional monitoring instrument is designed to only monitor one certain signal during a surgical procedure due to its structural limitation, incapable of monitoring multiple signals simultaneously. Detection of the intrarectal pressure and the external anal sphincter myoelectric recordings relies on multiple sets of invasive monitoring instruments that work separately, resulting in a large trauma as well as a high expense to the patient; in addition, conventional measurement approaches are incapable of simultaneously monitoring the intrarectal pressure and external anal sphincter myoelectric recordings, which leads to deteriorated operative safety and increased postoperative risks.

Specifically, the neuroelectrophysiological monitoring apparatus 200 comprises a rectal pressure measuring member, a myoelectricity measuring member, and a monitoring module 23. The rectal pressure measuring member is set in a patient's rectum and adaptable to measure the patient's intrarectal pressure; the myoelectricity measuring member is disposed adjacent to the rectal pressure measuring member and adapted to contact a mucous membrane on a surface of the external anal sphincter; contact between the myoelectricity measuring member and the mucous membrane on the surface of the external anal sphincter allows for measuring the external anal sphincter electromyographic activity; the monitoring module 23 is connected to the rectal pressure measuring member and the myoelectricity measuring member, respectively; the rectal pressure measuring member and the myoelectricity measuring member may transmit signals to the monitoring module 23, so that the monitoring module 23 monitors the patient's intrarectal pressure and external anal sphincter electromyographic activity based on the signals received.

In brief, with the pressure measuring member configured to measure a patient's intrarectal pressure, the myoelectricity measuring member configured to measure an external anal sphincter electromyographic activity, and the monitoring module 23 configured to monitor the intrarectal pressure and the external anal sphincter electromyographic activity, the neuroelectrophysiological monitoring apparatus 200 according to the disclosure is enabled to simultaneously measure the intrarectal pressure and the external anal sphincter electromyographic activity, eliminating a need to deploy multiple monitoring instruments, which improves operative safety and reduces postoperative risks,

According to one embodiment, the rectal pressure measuring member comprises a communication tube 211, an intrarectal expandable balloon 215, and a pressure transducing device. A medium may be stored in the communication tube 211, wherein the medium may be a liquid medicine such as physiological saline. One end of the communication tube 211 is provided with an intrarectal expandable balloon 215, and the opposite end of the communication tube 211 extends out of the patient's body. The communication tube 211 is adapted to deliver the medium from the external to the patient's body, or deliver the medium from the inside the patient's body to the external.

The intrarectal expandable balloon 215 is disposed at one end of the communication tube 211, and the intrarectal expandable balloon 215 may communicate with the communication tube 211, wherein the intrarectal expandable balloon 215 may be configured as a rubber element. The intrarectal expandable balloon 215 is selectively deformable to be tensioned in the patient's rectum. Specifically, the intrarectal expandable balloon 215 has a contracted state and an expanded state. When the intrarectal expandable balloon 215 is in the contracted state, the communication tube 211 may enter or be removed out of the patient's rectum; when the intrarectal expandable balloon 215 is in the expanded state, the outer peripheral wall of the intrarectal expandable balloon 215 abuts against the inside lining of the patient's rectum, whereby the communication tube 211 is securely anchored in the patient's rectum; when the inside lining of the rectum is contracted, the intrarectal expandable balloon 215 is deformed as the pressure changes, thereby transducing the intrarectal pressure signal.

The pressure transducing device has a testing end 213 and a signal output end, the testing end 213 being in communication with the communication tube 211 and configured to measure a pressure of the medium in the communication tube 211, wherein an interconnecting catheter 212 is provided between the testing end 213 and the communication tube 211, one end of the interconnecting catheter 212 being connected to the communication tube 211, the opposite end of the interconnecting catheter 212 being connected to the testing end 213, so that the testing end 213 communicates with the communication tube 211. In another embodiment of the disclosure, the interconnecting catheter 212 and the communication tube 211 may be unitarily formed. An on/off valve may be provided between the interconnecting catheter 212 and the communication tube 211, the on/off valve allowing for selective communication between the interconnecting catheter 212 and the communication tube 211.

The signal output end is connected to the monitoring module 23; the pressure transducing device may convert the pressure of the medium to an intrarectal pressure signal and transmit the intrarectal pressure signal to the monitoring module 23 via the signal output end, so that the monitoring module 23 monitors the intrarectal pressure of the patient based on the intrarectal pressure signal.

According to one embodiment of the disclosure, the opposite end of the communication tube 211 is provided with a medium injecting device 214, wherein the medium injecting device 214 may be configured as a syringe. The medium injecting device 214 may selectively inject the medium into the communicating device 211 so that the medium may be delivered into the intrarectal expandable balloon 215 via the communication tube 211, causing the intrarectal expandable balloon 215 to be expanded. An on/off valve may be provided between the medium injecting device 214 and the communication tube 211, the on/off valve allowing for selective communication between the medium injecting device 214 and the communication tube 211.

According to one embodiment of the disclosure, the neuroelectrophysiological monitoring apparatus 200 further comprises a three-way tube, wherein the three-way tube may be configured as a third regulating valve 24, the third regulating valve 24 having a first interface, a second interface, and a third interface which selectively communicate with each other, wherein the first interface is connected to the opposite end of the communication tube 211, and the second interface is connected to the testing end 213 of the pressure transducing device; here, it is to be noted that connection of the second interface to the interconnecting catheter 212 realizes further connection of the second interface to the testing end 213 of the pressure transducing device, the third interface being connected to the medium injecting device 214. Provision of the three-way regulating valve may reduce port multiplexing and plugging operations during monitoring, which facilitates a manipulator to pre-connect the monitoring apparatus, further reducing manipulation difficulty.

Specifically, when the first interface communicates with the third interface while the second interface is shut down, the communication tube 211 communicates with the medium injecting device 214, in which case the medium injecting device 214 may selectively inject the medium into the communication tube 211 so that the medium may be delivered into the patient's intrarectal expandable balloon 215 via the communication tube 211; when the first interface communicates with the second interface while the third interface is shut down, the communication tube 211 communicates with the interconnecting catheter 212, in which case the pressure transducing device may convert the pressure of the medium in the interconnecting catheter 212 to an intrarectal pressure signal and transmit the intrarectal pressure signal to the monitoring module 23 via the signal output end, so that the monitoring module 23 monitors the patient's intrarectal pressure based on the intrarectal pressure signal.

According to one embodiment of the disclosure, the myoelectricity measuring member is disposed at the outer periphery of the communication tube 211. Furthermore, when the communication tube 211 enters the patient's rectum, the myoelectricity measuring member may directly contact the mucous membrane on the surface of the external anal sphincter. Contact between the myoelectricity measuring member and the mucous membrane on the surface of the external anal sphincter allows for measuring the external anal sphincter electromyographic activity. The myoelectricity measuring member may be integrated at the outer periphery of the communication tube 211, ensuring that the neuroelectrophysiological monitoring apparatus 200 measures the intrarectal pressure and the external anal sphincter electromyographic activity simultaneously, thereby realizing simultaneous measurement of two parameters, which improves operative safety and reduces postoperative risks.

According to one embodiment of the disclosure, the myoelectricity measuring member comprises a plurality of electromyography electrodes 221, the plurality of electromyography electrodes 221 being arranged at intervals at the outer periphery of the communication tube 211; the plurality of electromyography electrodes 221 may be attached to the mucous membrane on the surface of the external anal sphincter to record the external anal sphincter electromyographic activity, and by arranging the plurality of electromyography electrodes 221 at intervals at the outer periphery of the communication tube 211, measurement accuracy and comprehensiveness may be further improved, thereby enhancing operative safety.

According to one embodiment of the disclosure, the myoelectricity measuring member comprises a plurality of electromyography electrodes 221, the plurality of electromyography electrodes 221 being arranged at intervals at the outer periphery of the communication tube 211; the plurality of electromyography electrodes 221 are configured to record the external anal sphincter electromyographic activity by touching the mucous membrane on the surface of the external anal sphincter; by arranging the plurality of electromyography electrodes 221 at intervals at the outer periphery of the communication tube 211, measurement accuracy and comprehensiveness may be further improved, thereby enhancing operative safety.

According to one embodiment of the disclosure, each electromyography electrode 221 is configured to have a stripped shape, an extending direction of each electromyography electrode 221 is consistent with an extending direction of the communication tube 211, the plurality of electromyography electrodes 221 being arranged at intervals at the outer periphery of the communication tube 211, wherein the plurality of electromyography electrodes 221 are arranged at intervals, respective electromyography electrodes 221 being insulated from each other, each electromyography electrode 221 being configured to record an electromyography signal of the external anal sphincter, respectively. Specifically, the number of the strip-shaped electromyography electrodes 1212 may be 2, 4, 6, 8, or more; the number of the strip-shaped electromyography electrodes 1212 may also be odd. By arranging the plurality of strip-shaped electrodes at intervals in the extending direction of the communication tube 211, at least the electromyography signals of the left and right-sides of the external anal sphincter may be monitored, respectively; in addition, the electromyography signals of the external anal sphincter corresponding to different positions of the outer periphery of the communication tube 211 may also be monitored, thereby realizing more accurate monitoring of the electromyographic activities at different positions of the external anal sphincter.

According to one embodiment of the disclosure, each electromyography electrode 221 is configured to have a ring shape and disposed surrounding the communication tube 211, the plurality of electromyography electrodes 221 being arranged at intervals in the extending direction of the communication tube 211, wherein the plurality of electromyography electrodes 221 are arranged at intervals, respective electromyography electrodes 221 being insulated from each other, each electromyography electrode 221 being configured to measure an electromyography signal of the external anal sphincter, respectively, enabling measurement of the electromyography signals at multiple positions of the external anal sphincter, whereby the electromyography electrodes 221 measures the electromyography signals of the external anal sphincters more accurately. By arranging the plurality of ring-shaped electromyography electrodes 1211 at intervals surrounding the communication tube 211, it is enabled to monitor the electromyography signals of the external anal sphincter corresponding to different depth positions in the extending direction of the communication tube 211, whereby the electromyographic activities at different positions of the external anal sphincter may be monitored more accurately.

According to one embodiment of the disclosure, the neuroelectrophysiological monitoring apparatus 200 further comprises an electromyography electrode connecting harness 222, the electromyography electrode connecting harness 222 being adapted to electrically connect the electromyography electrode 221 and the monitoring module 23, wherein the electromyography electrode connecting harness 222 is at least partially disposed inside or on the tube wall of the communication tube 211, one end of the electromyography electrode connecting harness 222 being connected to the plurality of electromyography electrodes 221, the opposite end of the electromyography electrode connecting harness 222 being connected to the monitoring module 23; the plurality of electromyography electrodes 221 may be independently and separately connected to the monitoring module 23 via the electromyography electrode connecting harness 222, so that the measured electromyography signals are transmitted to the monitoring module 23 via the electromyography electrode connecting harness 222.

According to one embodiment of the disclosure, a plurality of scale markers arranged at intervals in the extending direction of the communication tube 211 are provided on an outer peripheral wall of the communication tube 211. The scale markers are adapted to adjust the depth of the communication tube 211 inserted into the patient's anus, ensuring that the communication tube 211 is always disposed in a safe depth, thereby improving operative safety and reducing postoperative risks.

Hereinafter, a neuroelectrophysiological monitoring apparatus according to a third aspect of embodiments of the disclosure will be described, the neuroelectrophysiological monitoring apparatus being configured to measure a patient's intravesical pressure, intrarectal pressure, external urethra sphincter electromyographic activity, and external anal sphincter electromyographic activity.

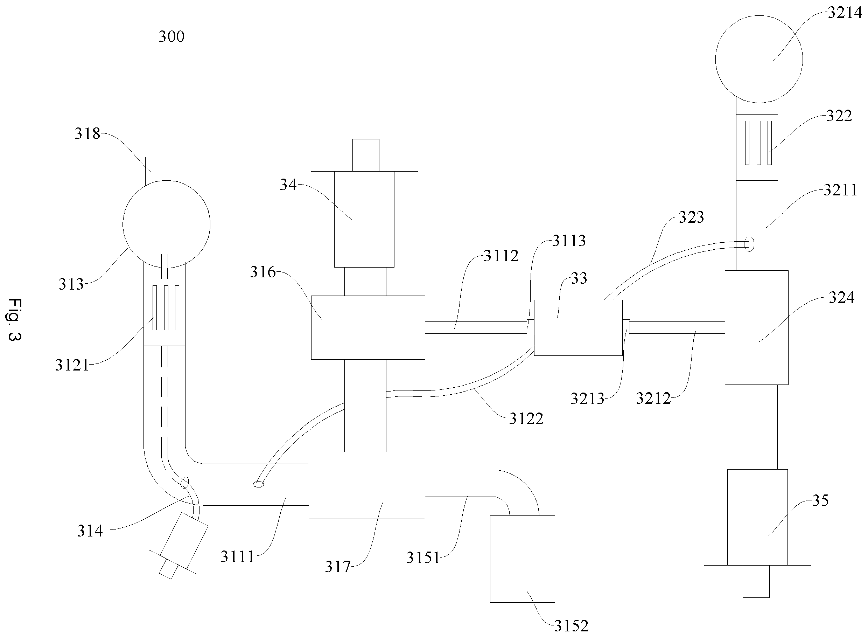

Hereinafter, referring to Figs. 3-5, a neuroelectrophysiological monitoring apparatus 300 according to embodiments of the disclosure is described, the neuroelectrophysiological monitoring apparatus 300 comprising a urethra testing device, a rectum testing device, and a monitoring module 33. The urethra testing device is provided with a bladder pressure measuring member and a first myoelectricity measuring member, the bladder pressure measuring member being set in a patient's bladder and adapted to measure the patient's intravesical pressure, the first myoelectricity measuring member being adapted to contact a mucous membrane on a surface of the external urethra sphincter to measure an external urethra sphincter electromyographic activity; the rectum testing device is provided with a rectal pressure measuring member and a second myoelectricity measuring member, the rectal pressure measuring member being set in the patient's rectum and adapted to measure the patient's intrarectal pressure, the second myoelectricity measuring member being adapted to contact a mucous membrane on a surface of the external anal sphincter to measure an external anal sphincter electromyographic activity; and the monitoring module 33 is electrically connected to the bladder pressure measuring member, the first myoelectricity measuring member, the rectal pressure measuring member, and the second myoelectricity measuring member, respectively.

At present, intraoperatively monitored signals in relevant techniques include an intravesical pressure, an intrarectal pressure, an external urethra sphincter electromyographic activity, and an external anal sphincter electromyographic activity. However, a conventional monitoring instrument is designed to only monitor one certain signal during a surgical procedure due to its structural limitation, incapable of monitoring multiple signals simultaneously. Detection of the intravesical pressure, the intrarectal pressure, the external urethra sphincter myoelectric recording, and the external anal sphincter myoelectric recording relies on multiple sets of invasive monitoring instruments that work separately, resulting in a large trauma as well as a high expense to a patient; in addition, conventional measurement approaches are incapable of simultaneously monitoring the intravesical pressure, the intrarectal pressure, the external urethra sphincter myoelectric recording, and the external anal sphincter myoelectric recording, which leads to deteriorated operative safety and increased postoperative risks.

Specifically, the neuroelectrophysiological monitoring apparatus 300 comprises a urethra testing device, a rectum testing device, and a monitoring module 33. The urethra testing device is provided with a bladder pressure measuring member and a first myoelectricity measuring member, the bladder pressure measuring member being set in the patient's bladder and adaptable to measure the patient's intravesical pressure, the first myoelectricity measuring member being disposed adjacent to the bladder pressure measuring member and adaptable to contact a mucous membrane on a surface of the external urethra sphincter. Contact between the first myoelectricity measuring member and the external urethra sphincter allows for measuring an external urethra sphincter electromyographic activity.

The rectal pressure measuring member is set in the patient's rectum and adaptable to measure the patient's intrarectal pressure; the second myoelectricity measuring member is set adjacent to the rectal pressure measuring member and adaptable to contact a mucous membrane on a surface of the external anal sphincter; contact between the second myoelectricity measuring member and the mucous membrane on the surface of the external anal sphincter allows for measuring an external anal sphincter electromyographic activity.

The monitoring module 33 is electrically connected to the bladder pressure measuring member, the first myoelectricity measuring member, the rectal pressure measuring member, and the second myoelectricity measuring member, respectively, the bladder pressure measuring member, the first myoelectricity measuring member, the rectal pressure measuring member, and the second myoelectricity measuring member being adapted to transmit signals to the monitoring module 33 so that the monitoring module 33 monitors the patient's intravesical pressure, intrarectal pressure, external urethra sphincter electromyographic activity, and external anal sphincter electromyographic activity based on the signals received.

In this embodiment, the urethra testing device may refer to the neuroelectrophysiological monitoring apparatus 100 according to a first aspect of embodiments, and the rectum testing device may refer to the neuroelectrophysiological monitoring apparatus 200 according to a second aspect of embodiments.

In brief, with the urethra testing device configured to measure the patient's intravesical pressure and external urethra sphincter electromyographic activity, the rectum testing device configured to measure the patient's intrarectal pressure and external anal sphincter electromyographic activity, and the monitoring module 33 configured to monitor the intravesical pressure, the intrarectal pressure, the external urethra sphincter electromyographic activity, and the external anal sphincter electromyographic activity, the neuroelectrophysiological monitoring apparatus 300 according to the disclosure is enabled to simultaneously monitor the intravesical pressure, the intrarectal pressure, the external urethra sphincter electromyographic activity, and the external anal sphincter electromyographic activity, eliminating a need to deploy multiple monitoring instruments, which improves integrity of the neuroelectrophysiological monitoring apparatus 300, improves operative safety, and reduces postoperative risks.

According to one embodiment of the disclosure, the neuroelectrophysiological monitoring apparatus 300 further comprises a first communication tube 3111 and a first pressure transducing device. A medium may be stored in the first communication tube 3111, wherein the medium may refer to a liquid such as physiological saline. One end of the first communication tube 3111 is open and set in the patient's bladder, and the opposite end of the first communication tube 3111 extends out of the patient's body. The first communication tube 3111 is adapted to deliver the medium, whereby the medium is delivered from the external to the patient's body, or delivered from the inside the patient's body to the external.

The first pressure transducing device has a first testing end 3113 and a first signal output end, the first testing end 3113 being in communication with the first communication tube 3111 and configured to measure a pressure of the medium in the communication tube, wherein a first interconnecting catheter 3112 is provided between the first testing end 3113 and the first communication tube 3111, one end of the first interconnecting catheter 3112 being connected to the first communication tube 3111, opposite end of the first interconnecting catheter 3112 being connected to the first testing end 3113, so that the first testing end 3113 communicates with the first communication tube 3111; and a diameter of the first interconnecting catheter 3112 is smaller than that of the communication tube, which ensures that the first interconnecting catheter 3112 may be fitted with a port of the first testing end 3113. In another embodiment of the disclosure, the first interconnecting catheter 3112 and the first communication tube 3111 may be unitarily formed. An on/off valve may be provided between the first interconnecting catheter 3112 and the first communication tube 3111, the on/off valve allowing for selective communication between the first interconnecting catheter 3112 and the first communication tube 3111.

The first signal output end is connected to the monitoring module 33; the first pressure transducing device may convert the pressure of the medium to an intravesical pressure signal and transmit the intravesical pressure signal to the monitoring module 33 via the signal output end, so that the monitoring module 33 monitors the intravesical pressure of the patient based on the intravesical pressure signal.

According to one embodiment of the disclosure, the opposite end of the first communication tube 3111 is provided with a first medium injecting device 34, wherein the first medium injecting device 34 may be configured as a syringe. The first medium injecting device 34 may selectively inject a medium into the first communicating device 3111 so that the medium may be delivered into the patient's bladder via the first communication tube 311. An on/off valve may be provided between the first medium injecting device 34 and the first communication tube 3111, the on/off valve allowing for selective communication between the first medium injecting device 34 and the first communication tube 3111.

According to one embodiment of the disclosure, the neuroelectrophysiological monitoring apparatus 300 further comprises a fourth regulating valve 316, wherein the fourth regulating valve 316 may be configured as a three-way regulating valve, the fourth regulating valve 316 comprising a first port, a second port, and a third port, the first port, the second port, and the third port being in one-to-one communication with the first communication tube 3111, the first interconnecting catheter 3112, and the first medium injecting device 34, respectively. Provision of the three-way regulating valve may reduce port multiplexing and plugging operations during monitoring, which facilitates a manipulator to pre-connect the monitoring apparatus, further reducing manipulation difficulty.

When the first port communicates with the third port while the second port is shut down, the first communication tube 3111 communicates with the first medium injecting device 34, in which case the first medium injecting device 34 may selectively inject a medium into the first communication tube 3111 so that the medium may be delivered into the patient's bladder via the first communication tube 3111; when the first port communicates with the second port while the third port is shut down, the first communication tube 3111 communicates with the first interconnecting catheter 3112, in which case the pressure transducing device may convert the pressure of the medium in the first interconnecting catheter 3112 to an intravesical pressure signal and transmit the intravesical pressure signal to the monitoring module 33 via the signal output end, so that the monitoring module 33 monitors the patient's intravesical pressure based on the intravesical pressure signal.

In another embodiment of the disclosure, the first interconnecting catheter 3112 or the first medium injecting device 114 may be directly connected to the first communication tube 3111, thereby eliminating configuration of the fourth regulating valve 316.

According to one embodiment of the disclosure, the first myoelectricity measuring member is set at an outer periphery of the first communication tube 3111. Furthermore, when the first communication tube 3111 enters the patient's bladder, the first myoelectricity measuring member may directly contact a mucous membrane on a surface of the external urethra sphincter. Contact between the myoelectricity measuring member and the mucous membrane on the surface of the external urethra sphincter allows for measuring an external urethra sphincter electromyographic activity. The first myoelectricity measuring member may be integrated at the outer periphery of the first communication tube 3111, ensuring that the neuroelectrophysiological monitoring apparatus 300 measures the intravesical pressure and the external urethra sphincter electromyographic activity simultaneously, thereby realizing simultaneous measurement of two parameters, which improves operative safety and reduces postoperative risks.

According to one embodiment of the disclosure, the first myoelectricity measuring member comprises a plurality of first electromyography electrodes 3121, the plurality of first electromyography electrodes 3121 being arranged at intervals at the outer periphery of the first communication tube 3111; the plurality of first electromyography electrodes 3121 may record the external urethra sphincter electromyographic activity by touching the mucous membrane on the surface of the external urethra sphincter; by arranging the plurality of first electromyography electrodes 3121 at intervals at the outer periphery of the first communication tube 3111, measurement accuracy and comprehensiveness may be further improved, thereby enhancing operative safety.

According to one embodiment of the disclosure, each first electromyography electrode 3121 is configured to have a stripped shape, an extending direction of each first electromyography electrode 3121 is consistent with an extending direction of the first communication tube 3111, the plurality of first electromyography electrodes 3121 being arranged at intervals at the outer periphery of the first communication tube 3111, wherein a length of individual strip-shaped first electromyography electrodes 3121 may be set between 2 cm and 3 cm, the length being preferably 2.5 cm; the plurality of first electromyography electrodes 3121 are arranged at intervals, respective first electromyography electrodes 3121 being insulated from each other, each first electromyography electrode 3121 being configured to measure an electromyography signal of the external urethra sphincter, respectively. Specifically, the number of the strip-shaped first electromyography electrodes 3121 may be 2, 4, 6, 8, or more; the number of the strip-shaped first electromyography electrodes 3121 may also be odd. By arranging the plurality of strip-shaped electrodes at intervals in the extending direction of the communication tube 3111, at least the electromyography signals of the left and right sides of the external urethra sphincter may be monitored, respectively; in addition, the electromyography signals of the external urethra sphincter corresponding to different positions of the outer periphery of the communication tube 3111 may also be monitored, thereby monitoring the electromyographic activities at different positions of the external urethra sphincter more accurately.

According to one embodiment of the disclosure, each first electromyography electrode 3121 is configured to have a ring shape and disposed surrounding the communication tube, the plurality of first electromyography electrodes 3121 being arranged at intervals in the extending direction of the first communication tube 3111, wherein a distribution width of the ring-shaped first electromyography electrodes 3121 may be set between 2 cm and 3 cm, the distribution width of the ring-shaped first electromyography electrodes 3121 being preferably 2.5 cm; the plurality of electromyography electrodes 3121 are arranged at intervals, respective first electromyography electrodes 3121 being insulated from each other, each first electromyography electrode 3121 being configured to measure an electromyography signal of the external urethra sphincter, respectively, enabling measurement of the electromyography signals at multiple positions of the external urethra sphincter, whereby the electromyography electrodes measure the electromyography signals of the external urethra sphincter more accurately. By arranging a plurality of ring-shaped electrodes at intervals surrounding the communication tube 3111, it is enabled to monitor the electromyography signals of the external urethra sphincter at corresponding different depth positions in the extending direction of the communication tube 3111, whereby the electromyographic activities at different positions of the external urethra sphincter may be monitored more accurately.

In a neuroelectrophysiological monitoring apparatus 300 applicable to an adult male patient, the distance between the upper end of each electromyography electrode 3121 and the start of the patient's bladder neck may range from 2 cm to 5 cm, the distance being preferably 3.5 cm. In a neuroelectrophysiological monitoring apparatus 300 applicable to an adult female patient, the distance between the upper end of each electromyography electrode 3121 and the start of the patient's bladder neck may range from 0.5 cm to 3 cm, the distance being being preferably 1.5 cm. In a neuroelectrophysiological monitoring apparatus 300 applicable to an underage patient, the distance between the upper end of each electromyography electrode 3121 and the start of the patient's bladder neck is smaller than the distance between the upper end of each electromyography electrode 3121 and the start of the patient's bladder neck in the neuroelectrophysiological monitoring apparatus 300 applicable to an adult male patient or adult female patient.

According to one embodiment of the disclosure, the neuroelectrophysiological monitoring apparatus 300 further comprises a first electromyography electrode connecting harness 3122, the first electromyography electrode connecting harness 3122 being adapted to electrically connect the first electromyography electrodes 3121 and the monitoring module 33. Specifically, the first electromyography electrode connecting harness 3122 is at least partially disposed inside or on the tube wall of the first communication tube 3111, one end of the first electromyography electrode connecting harness 3122 being connected to the plurality of first electromyography electrodes 3121, the opposite end of the first electromyography electrode connecting harness 3122 being connected to the monitoring module 33; the plurality of first electromyography electrodes 3121 may be separately and independently connected to the monitoring module 33 via the first electromyography electrode connecting harness 3122 so as to transmit the measured first electromyography signals to the monitoring module 33 via the first electromyography electrode connecting harness 3122.

According to one embodiment of the disclosure, the neuroelectrophysiological monitoring apparatus 300 further comprises a first communication tube 3111 anchoring device, the first communication tube 3111 anchoring device being disposed at one end of the first communication tube 3111, wherein the first communication tube 3111 anchoring device is configured as a rubber element, and the first communication tube 3111 anchoring device is selectively deformed to be tensioned in the patient's bladder. Specifically, the first communication tube 3111 anchoring device has a contracted state and an expanded state. When the first communication tube 3111 anchoring device is in the contracted state, the first communication tube 3111 may enter or be removed out of the patient's bladder, so that when the first communication tube 3111 anchoring device is in the expanded state, the first communication tube 3111 is anchored in the patient's bladder.

An expandable cavity is formed inside the first communication tube 3111 anchoring device, the expandable cavity being selectively expanded or contracted. The expandable cavity communicates with a syringe connection tube 314, the syringe connection tube 314 being adapted to inject a medium in the expandable cavity to control deformation of the communication tube anchoring device 313, wherein the medium may refer to a liquid or a gas; the syringe connection tube 314 allows for expansion of the expandable cavity by injecting the medium into the expandable cavity, so that the communication tube anchoring device 313 securely contacts the inside lining of the patient's bladder.

According to one embodiment of the disclosure, the syringe connection tube 314 is at least partially received in the communication tube, which reduces footprint of the syringe connection tube 314 and improves integrity of the syringe connection tube 314.

According to one embodiment of the disclosure, the neuroelectrophyiological monitoring apparatus 300 further comprises a urethra catheter 3151, one end of the urethra catheter 3151 being selectively conducted with the communication tube, opposite end of the urethra catheter 3151 being provided with a urine collection bag 3152, the urine collection bag 3152 being configured to collect residual urine or medium in the patient's bladder. Specifically, the neuroelectrophysiological monitoring apparatus 300 further comprises an intravesical liquid outlet 318, the intravesical liquid outlet 318 being adapted to drain the residual urine or medium out of the bladder; the residual urine or medium in the bladder flows into the urethra catheter 3151 along the communication tube and is then delivered into the urine collection bag 3152 via the urethra catheter 3151 till the operation ends.

According to one embodiment of the disclosure, the neuroelectrophysiological monitoring apparatus 300 further comprises a fifth regulating valve 317, the fifth regulating valve 317 comprising a fourth port, a fifth port, and a sixth port, the fourth port, the fifth port, and the sixth port being set in one-to-one correspondence with a front segment of the first communication tube 3111, a rear segment of the first communication tube 3111, and the urethra catheter 3151. Provision of the three-way regulating valve may reduce port multiplexing and plugging operations during monitoring, which facilitates a manipulator to pre-connect the monitoring apparatus, further reducing manipulation difficulty.

When the fourth port and the fifth port communicate while the sixth port is shut down, the front segment of the first communication tube 3111 is conducted with the rear segment of the first communication tube 3111, in which case the first medium injecting device 34 may inject the medium into the patient's bladder to facilitate monitoring of the patient's intravesical pressure. When the fourth port communicates with the sixth port while the fifth port is shut down, the front segment of the first communication tube 3111 is conducted with the urethra catheter 3151, in which case the residual urine or medium in the patient's bladder flows into the urethra catheter 3151 along the first communication tube 3111 and is then delivered into the urine collection bag 3152 via the urethra catheter 3151 till the operation ends.

In the neuroelectrophysiological monitoring apparatus 300 according to the disclosure, the rectal pressure measuring member comprises a second communication tube 3211, an intrarectal expandable balloon 3214, and a second pressure transducing device. A medium may be stored in the second communication tube 3211, wherein the medium may be a liquid such as physiological saline. One end of the second communication tube 3211 is disposed in the patient's rectum, and the opposite end of the second communication tube 3211 extends out of the patient's body. The second communication tube 3211 is adapted to deliver the medium, whereby the medium is delivered from the external into the patient's body, or delivered from inside the patient's body to the outside.

The intrarectal expandable balloon 3214 is disposed at one end of the second communication tube 3211, and the intrarectal expandable balloon 3214 may communicate with the second communication tube 3211, wherein the intrarectal expandable balloon 3214 may be configured as a rubber element. The intrarectal expandable balloon 3214 is selectively deformable to be tensioned in the patient's rectum. Specifically, the intrarectal expandable balloon 3214 has a contracted state and an expanded state. when the intrarectal expandable balloon 3214 is in the contracted state, the second communication tube 3211 may enter or be removed out of the patient's rectum; when the intrarectal expandable balloon 3214 is in the expanded state, the outer peripheral wall of the intrarectal expandable balloon 3214 abuts against the inside lining of the patient's rectum, whereby the second communication tube 3211 is anchored in the patient's rectum; when the inside lining of the rectum is contracted, the intrarectal expandable balloon 3214 is deformed as the pressure changes, whereby the intrarectal pressure signals are conducted.

The second pressure transducing device has a second testing end 3213 and a second signal output end, the second testing end 3213 communicating with the second communication tube 3211, the second testing end 3213 being configured to measure a pressure of the medium in the second communication tube 3211, wherein a second interconnecting catheter 3212 is provided between the second testing end 3213 and the second communication tube 3211, one end of the second interconnecting catheter 3212 being connected to the second communication tube 3211, the opposite end of the second interconnecting catheter 3212 being connected to the second testing end 3213, whereby the second testing end 3213 communicates with the second communication tube 3211; a diameter of the second interconnecting catheter 3212 is smaller than that of the second communication tube 3211, ensuring that the second interconnecting catheter 3212 may fit with the port of the second testing end 3213. In another embodiment of the disclosure, the second interconnecting catheter 3212 and the second communication tube 3211 may be unitarily configured. An on/off valve may be set between the second interconnecting catheter 3212 and the second communication tube 3211; provision of the on/off valve enables selective communication between the second interconnecting catheter 3212 and the second communication tube 3211.

According to one embodiment of the disclosure, the opposite end of the second communication tube 3211 is provided with a second medium injecting device 35, wherein the second medium injecting device 35 may be configured as a syringe. The second medium injecting device 35 selectively injects the medium into the second communication tube 3211, so that the medium may flow into the intrarectal expandable balloon 3214 via the second communicating tube 3211. An on/off valve may be provided between the second medium injecting device 35 and the second communication tube 3211; provision of the on/off valve enables selective communication between the second medium injecting device 35 and the second communication tube 3211.

According to one embodiment of the disclosure, the neuroelectrophysiological monitoring apparatus 300 further comprises a three-way tube, wherein the three-way tube may be configured as a sixth regulating valve 324, the sixth regulating valve 324 having a first interface, a second interface, and a third interface which selectively communicate with each other, wherein the first interface is connected to the opposite end of the second communication tube 3211, and the second interface is connected to the second testing end 3213 of the second pressure transducing device. Here, it is noted that connection of the second interface to the second interconnecting catheter 3212 further realizes connection of the second interface to the second testing end 3213 of the second pressure transducing device, the third interface being connected to the second medium injecting device 35. Provision of the the three-way regulating valve may reduce port multiplexing and plugging operations during monitoring, which facilitates a manipulator to pre-connect the monitoring apparatus, further reducing the manipulation difficulty.

Specifically, when the first interface communicates with the third interface while the second interface is shut down, the second communication tube 3211 communicates with the second medium injecting device 35, in which case the second medium injecting device 35 may selectively inject the medium into the second communication tube 3211, so that the medium may be delivered into the intrarectal expandable balloon 3214 via the second communication tube 3211; when the first interface communicates with the second interface while the third interface is shut down, the second communication tube 3211 communicates with the second interconnecting catheter 3212, in which case the pressure transducing device may convert the pressure of the medium in the second interconnecting catheter 3212 into an intrarectal pressure signal and transmit the intrarectal pressure signal to the monitoring module 33 via the signal output end, so that the monitoring module 33 monitors the patient's intrarectal pressure based on the intrarectal pressure signal.

According to one embodiment of the disclosure, the second myoelectricity measuring member is disposed at an outer periphery of the second communication tube 3211; furthermore, when the second communication tube 3211 enters the patient's rectum, the second myoelectricity measuring member may directly contact the mucous membrane on the surface of the external anal sphincter; contact between the myoelectricity measuring member and the mucous membrane on the surface of the external anal sphincter allows for measuring a electromyographic activity of the external anal sphincter; the second myoelectricity measuring member may be integrated at the outer periphery of the second communication tube 3211, ensuring that the neuroelectrophysiological monitoring apparatus 300 simultaneously monitors the intrarectal pressure and the external anal sphincter electromyographic activity, thereby realizing simultaneous measurement of two parameters, with improved operative safety and reduced postoperative risks.

According to one embodiment of the disclosure, the second myoelectricity measuring member comprises a plurality of second electromyography electrodes 322, the plurality of second electromyography electrodes 322 being arranged at intervals at the outer periphery of the second communication tube 3211; the plurality of second electromyography electrodes 322 may obtain external anal sphincter electromyographic activities by touching the external anal sphincter; by arranging the plurality of second electromyography electrodes 322 at intervals at the outer periphery of the second communication tube 3211, measurement accuracy and comprehensiveness may be further improved, thereby enhancing operative safety.

According to one embodiment of the disclosure, each second electromyography electrode 322 is configured to have a stripped shape, an extending direction of each second electromyography electrode 322 being consistent with an extending direction of the second communication tube 3211, the plurality of second electromyography electrodes 322 being arranged at intervals along the outer periphery of the second communication tube 3211, wherein the plurality of second electromyography electrodes 322 are arranged at intervals, respective second electromyography electrodes 322 being insulated from each other, each second electromyography electrode 322 being configured to measure an electromyography signal of the external anal sphincter, respectively. Specifically, the number of the strip-shaped second electromyography electrodes 322 may be 2, 4, 6, 8, or more; the number of the strip-shaped second electromyography electrodes 322 may also be odd. By arranging the plurality of strip-shaped electrodes at intervals in the extending direction of the communication tube 3211, at least the electromyography signals of the left and right sides of the external anal sphincter may be monitored, respectively; and the electromyography signals of the external anal sphincter corresponding to different positions in the circumferential direction of the communication tube 3211 may also be monitored, thereby realizing more accurate monitoring of the electromyographic activities of different positions of the external anal sphincter.

According to one embodiment of the disclosure, each second electromyography electrode 322 is configured to have a ring shape and disposed surrounding the communication tube, the plurality of second electromyography electrodes 322 being arranged at intervals in the extending direction of the second communication tube 3211, wherein the plurality of second electromyography electrodes 322 are arranged at intervals, respective second electromyography electrodes 322 being insulated from each other, each second electromyography electrode 322 being configured to measure an electromyography signal of the external anal sphincter, respectively, enabling measurement of the electromyography signals at multiple positions of the external anal sphincter, whereby the electromyography electrodes measure the electromyography signals of the external anal sphincter more accurately. By providing the plurality of ring-shaped electrodes at intervals surrounding the communication tube 3211, it is enabled to monitor the electromyography signals of the external anal sphincter corresponding to different depth positions in the extending direction of the communication tube 3211, so that the electromyographic activities of different positions of the external anal sphincter are measured more accurately.

According to one embodiment of the disclosure, the neuroelectrophysiological monitoring apparatus 300 further comprises a second electromyography electrode connecting harness 323, the second electromyography electrode connecting harness 323 being adapted to electrically connect the second electromyography electrode 322 and the monitoring module 33, wherein the second electromyography electrode connecting harness 323 is at least partially disposed inside or on the tube wall of the second communication tube 3211, one end of the second electromyography electrode connecting harness 323 being connected to the plurality of second electromyography electrodes 322, opposite end of the second electromyography electrode connecting harness 323 being connected to the monitoring module 33; the plurality of second electromyography electrodes 322 may be connected separately and independently to the monitoring module 33 via the second electromyography electrode connecting harness 323 so as to transmit the measured second electromyography signals to the monitoring module 33 via the second electromyography electrode connecting harness 323.

According to one embodiment of the disclosure, a plurality of scale markers arranged at intervals in the extending direction of the first communication tube 3111 are provided at the outer peripheral wall of the first communication tube 3111, the scale markers being adapted to adjust the depth of the first communication tube 3111 inserted into the patient's urethra, which ensures that the first communication tube 3111 is always in a safe depth, thereby improving operative safety and reducing postoperative risks. **In** addition, a plurality of scale markers arranged at intervals in the extending direction of the second communication tube 3211 are provided on the outer peripheral wall of the second communication tube 3211, the scale markers being adapted to adjust the depth of the second communication tube 3211 inserted into the patient's anus, which ensures that the second communication tube 3211 is always in a safe depth, thereby improving operative safety and reducing postoperative risks.

In the neuroelectrophysiological monitoring apparatus 300 according to the disclosure, the urethra testing device is configured to measure the intravesical pressure and external urethra sphincter electromyographic activity, and the rectum testing device is configured to measure the intrarectal pressure and the external anal sphincter electromyographic activity. Therefore, the neuroelectrophysiological monitoring apparatus 300 may simultaneously measure a patient's intravesical pressure, intrarectal pressure, external urethra sphincter electromyographic activity, and external anal sphincter electromyographic activity simultaneously during a surgical procedure; moreover, the neuroelectrophysiological apparatus 300 integrates the urethra testing device and the rectum testing device in the same apparatus, eliminating a need of deploying multiple monitoring instruments during a surgical procedure, which improves integrity of the neuroelectrophysiological monitoring apparatus 300, improves operative safety, and reduces postoperative risks.

In the description of the disclosure, it is noted that the orientational or positional relationships indicated by terms such as "center," "longitudinal," "transverse," "length," "width," "thickness," "upper," "lower," "front," "rear," "left," "right," "vertical," "horizontal," "top," "bottom," "inner," "outer," "clockwise," "counterclockwise," "axial," "radial," and "peripheral" refer to those orientational or positional relationships illustrated in the drawings, which are only intended to facilitate describing the disclosure and simplify the description, rather than indicating or implying that the referred to devices or elements must have such specific orientations, or be configured and operated with such specific orientations; therefore, they should not be construed as limitations to the disclosure.

In the description of the disclosure, the terms "first feature" and "second feature" may include one or more such features.

In the description of the disclosure, the term "plurality" means two or more.

In the description of the disclosure, a first feature being "above" or "below" a second feature may refer to direct contact between the first feature and the second feature, or an indirect contact between the first feature and the second feature via an intermediate feature.

In the description of the disclosure, a first feature being "above," "over," or "on top of" a second feature refers to the first feature being directly perpendicular to or not in direct vertical line with the second feature, or only indicates that a horizontal level of the first feature is higher than that of the second feature.

In the description of the disclosure, reference to "one embodiment," "some embodiments," "schematic embodiments," "an example," "a specific example," or "some examples" intend to mean that a specific feature, structure, material or characteristic described in conjunction with the embodiment or example is included in at least one embodiment of example of the disclosure. In the present description, schematic references to the terms noted *supra* do not necessarily refer to same embodiment or example. Moreover, the specific features, structures, materials or characteristics as described may be combined appropriately in any one or more embodiments or examples.

## Claims

1. A neuroelectrophysiological monitoring apparatus (300), comprising:
a urethra testing device, the urethra testing device being provided with a bladder pressure measuring member and a first myoelectricity measuring member, the bladder pressure measuring member configured for being set in a patient's bladder and adapted to measure the patient's intravesical pressure, the first myoelectricity measuring member being adapted to contact an external urethra sphincter to measure an external urethra sphincter electromyographic activity;
a rectum testing device, the rectum testing device being provided with a rectal pressure measuring member and a second myoelectricity measuring member, the rectal pressure measuring member configured for being set in the patient's rectum and adapted to measure the patient's intrarectal pressure, the second myoelectricity measuring member being adapted to contact an external anal sphincter to measure an external anal sphincter electromyographic activity; and
a monitoring module (33), the monitoring module (33) being electrically connected to the bladder pressure measuring member, the first myoelectricity measuring member, the rectal pressure measuring member, and the second myoelectricity measuring member, respectively so as to monitor an electrophysiological activity of pudendal nerve.

2. The neuroelectrophysiological monitoring apparatus (300) according to claim 1, further comprising:
a first communication tube (3111), the first communication tube (3111) being adapted to deliver a medium, one end of the first communication tube (3111) being open and set in the patient's bladder, opposite end of the first communication tube (3111) extending outside the patient's body; and
a first pressure transducing device, the first pressure transducing device having a first testing end (3113) and a first signal output end, the first testing end (3113) communicating with the first communication tube (3111) and being adapted to measure a pressure of the medium in the first communication tube (3111), the first signal output end being in communicative connection to the monitoring module (33), the first pressure transducing device being adapted to convert the pressure of the medium in the first communication tube (3111) to an intravesical pressure signal and to transmit the intravesical pressure signal to the monitoring module (33) via the first signal output end.

3. The neuroelectrophysiological monitoring apparatus (300) according to claim 2, wherein the first myoelectricity measuring member is disposed at an outer periphery of the first communication tube (3111), wherein the first myoelectricity measuring member is formed of a plurality of first electromyography electrodes (3121), the plurality of first electromyography electrodes (3121) being arranged at intervals in an extending direction of the first communication tube (3111) or in a circumferential direction of the first communication tube (3111).

4. The neuroelectrophysiological monitoring apparatus (300) according to claim 2, further comprising:
a first communication tube anchoring device (313), the first communication tube anchoring device (313) being disposed at one end of the first communication tube (3111), the first communication tube anchoring device (313) configured to being selectively deformed to be tensioned in the patient's bladder, an expandable cavity being formed inside the first communication tube anchoring device (313), the expandable cavity communicating with a syringe connection tube (314) so that the medium is injected into the expandable cavity to control deformation of the communication tube anchoring device (313).

5. The neuroelectrophysiological monitoring apparatus (300) according to claim 2, wherein the rectal pressure measuring member comprises:
a second communication tube (3211), the second communication tube (3211) being adapted to deliver the medium, one end of the second communication tube (3211) being configured to being set in the patient's rectum, opposite end of the second communication tube (3211) extending outside the patient's body;
an intrarectal expandable balloon, the intrarectal expandable balloon being disposed at one end of the second communication tube (3211) and communicating with the second communication tube (3211), an outer peripheral wall of the intrarectal expandable balloon configured to being abutting against an inside lining of the patient's rectum; and
a second pressure transducing device, the second pressure transducing device having a second testing end and a second signal output end, the second testing end communicating with the second communication tube (3211) and being adapted to measure a pressure of the medium in the second communication tube (3211), the second signal output end being in communicative connection to the monitoring module (33), the second pressure transducing device being adapted to convert the pressure of the medium to an intrarectal pressure signal and transmit the intrarectal pressure signal to the monitoring module via the second signal output end.

6. The neuroelectrophysiological monitoring apparatus (300) according to claim 5, wherein the opposite end of the second communication tube (3211) is adaptable to be connected to the medium injecting device, the medium injecting device selectively injecting the medium into the second communication tube (3211)
wherein the neuroelectrophysiological monitoring apparatus (300) further comprising: a three-way tube, the three-way tube having a first interface, a second interface, and a third interface which selectively communicate with each other, the first interface being connected to the opposite end of the second communication tube (3211), the second interface being connected to the second testing end of the second pressure transducing device, the third interface being connected to the medium injecting device.

7. The neuroelectrophysiological monitoring apparatus (300) according to claim 6, wherein the second myoelectricity measuring member is disposed at an outer periphery of the second communication tube (3211), wherein the second myoelectricity measuring member is formed of a plurality of second electromyography electrodes, the plurality of second electromyography electrodes being arranged at intervals in an extending direction of the second communication tube (3211) or in a circumferential direction of the first communication tube (3111).

8. The neuroelectrophysiological monitoring apparatus (300) according to claim 7, wherein a plurality of scale markers arranged at intervals in the extending direction of the second communication tube (3211) are provided on an outer peripheral wall of the second communication tube (3211).

## Patentansprüche

1. Neuroelektrophysiologisches Überwachungsgerät (300), umfassend:
eine Harnröhrentestvorrichtung, wobei die Harnröhrentestvorrichtung mit einem Blasendruckmessglied und einem ersten Myoelektrizitätsmessglied versehen ist, wobei das Blasendruckmessglied dazu eingerichtet ist, in der Blase eines Patienten angeordnet zu werden und dazu geeignet ist, den intravesikalen Druck des Patienten zu messen, wobei das erste Myoelektrizitätsmessglied dazu geeignet ist, mit einem äußeren Harnröhrenschließmuskel in Kontakt zu stehen, um eine Elektromyografieaktivität des äußeren Harnröhrenschließmuskels zu messen;
eine Rektumtestvorrichtung, wobei die Rektumtestvorrichtung mit einem Rektaldruckmessglied und einem zweiten Myoelektrizitätsmessglied versehen ist, wobei das Rektaldruckmessglied dazu eingerichtet ist, im Rektum des Patienten angeordnet zu werden und dazu geeignet ist, den intrarektalen Druck des Patienten zu messen, wobei das zweite Myoelektrizitätsmessglied dazu geeignet ist, mit einem äußeren Afterschließmuskel in Kontakt zu stehen, um eine Elektromyografieaktivität des äußeren Afterschließmuskels zu messen; und
ein Überwachungsmodul (33), wobei das Überwachungsmodul (33) elektrisch jeweils mit dem Blasendruckmessglied, dem ersten Myoelektrizitätsmessglied, dem Rektaldruckmessglied und dem zweiten Myoelektrizitätsmessglied verbunden ist, um eine elektrophysiologische Aktivität des Nervus pudendus zu überwachen.

2. Neuroelektrophysiologisches Überwachungsgerät (300) nach Anspruch 1, ferner umfassend:
ein erstes Kommunikationsrohr (3111), wobei das erste Kommunikationsrohr (3111) dazu geeignet ist, ein Medium zu fördern, wobei ein Ende des ersten Kommunikationsrohrs (3111) offen ist und in der Blase des Patienten angeordnet wird, und das gegenüberliegende Ende des ersten Kommunikationsrohrs (3111) außerhalb des Körpers des Patienten verläuft; und
eine erste Druckumwandlungsvorrichtung, wobei die erste Druckumwandlungsvorrichtung ein erstes Testende (3113) und ein erstes Signalausgangsende aufweist, wobei das erste Testende (3113) mit dem ersten Kommunikationsrohr (3111) kommuniziert und dazu geeignet ist, einen Druck des Mediums im ersten Kommunikationsrohr (3111) zu messen, wobei das erste Signalausgangsende mit dem Überwachungsmodul (33) kommunikationstechnisch verbunden ist, wobei die erste Druckumwandlungsvorrichtung dazu geeignet ist, den Druck des Mediums im ersten Kommunikationsrohr (3111) in ein intravesikales Drucksignal umzuwandeln und das intravesikale Drucksignal über das erste Signalausgangsende an das Überwachungsmodul (33) zu übertragen.

3. Neuroelektrophysiologisches Überwachungsgerät (300) nach Anspruch 2, wobei das erste Myoelektrizitätsmessglied an einem Außenumfang des ersten Kommunikationsrohrs (3111) angeordnet ist, wobei das erste Myoelektrizitätsmessglied aus einer Vielzahl von ersten Elektromyografieelektroden (3121) besteht, wobei die Vielzahl von ersten Elektromyografieelektroden (3121) in Längsrichtung des ersten Kommunikationsrohrs (3111) oder in Umfangsrichtung des ersten Kommunikationsrohrs (3111) mit Abstand zueinander angeordnet sind.

4. Neuroelektrophysiologisches Überwachungsgerät (300) nach Anspruch 2, ferner umfassend:
eine erste Verankerungsvorrichtung für das Kommunikationsrohr (313), wobei die erste Verankerungsvorrichtung für das Kommunikationsrohr (313) an einem Ende des ersten Kommunikationsrohrs (3111) angeordnet ist, wobei die erste Verankerungsvorrichtung für das Kommunikationsrohr (313) dazu eingerichtet ist, selektiv verformt zu werden, um in der Blase des Patienten gespannt zu werden, wobei in der ersten Verankerungsvorrichtung für das Kommunikationsrohr (313) ein expandierbarer Hohlraum gebildet ist, wobei der expandierbare Hohlraum mit einem Spritzenverbindungsrohr (314) kommuniziert, sodass das Medium in den expandierbaren Hohlraum eingespritzt wird, um die Verformung der Verankerungsvorrichtung für das Kommunikationsrohr (313) zu steuern.

5. Neuroelektrophysiologisches Überwachungsgerät (300) nach Anspruch 2, wobei das Rektaldruckmessglied umfasst:
ein zweites Kommunikationsrohr (3211), wobei das zweite Kommunikationsrohr (3211) dazu geeignet ist, das Medium zu fördern, wobei ein Ende des zweiten Kommunikationsrohrs (3211) dazu eingerichtet ist, im Rektum des Patienten angeordnet zu werden, wobei das gegenüberliegende Ende des zweiten Kommunikationsrohrs (3211) außerhalb des Körpers des Patienten verläuft;
einen intrarektalen expandierbaren Ballon, wobei der intrarektale expandierbare Ballon an einem Ende des zweiten Kommunikationsrohrs (3211) angeordnet ist und mit dem zweiten Kommunikationsrohr (3211) kommuniziert, wobei eine Außenwand des intrarektalen expandierbaren Ballons dazu eingerichtet ist, an einer Innenauskleidung des Rektums des Patienten anzuliegen; und
eine zweite Druckumwandlungsvorrichtung, wobei die zweite Druckumwandlungsvorrichtung ein zweites Testende und ein zweites Signalausgangsende aufweist, wobei das zweite Testende mit dem zweiten Kommunikationsrohr (3211) kommuniziert und dazu eingerichtet ist, den Druck des Mediums im zweiten Kommunikationsrohr (3211) zu messen, wobei das zweite Signalausgangsende mit dem Überwachungsmodul kommunikationstechnisch verbunden ist, wobei die zweite Druckumwandlungsvorrichtung dazu eingerichtet ist, den Druck des Mediums in ein intrarektales Drucksignal umzuwandeln und das intrarektale Drucksignal über das zweite Signalausgangsende an das Überwachungsmodul zu übertragen.

6. Neuroelektrophysiologisches Überwachungsgerät (300) nach Anspruch 5, wobei das gegenüberliegende Ende des zweiten Kommunikationsrohrs (3211) dazu eingerichtet ist, mit einer Mediumseinspritzvorrichtung verbunden zu werden, wobei die Mediumseinspritzvorrichtung selektiv Medium in das zweite Kommunikationsrohr (3211) einspritzt,
wobei das neuroelektrophysiologische Überwachungsgerät (300) ferner umfasst: ein Dreiwegerohr, wobei das Dreiwegerohr eine erste Schnittstelle, eine zweite Schnittstelle und eine dritte Schnittstelle aufweist, die selektiv miteinander kommunizieren, wobei die erste Schnittstelle mit dem gegenüberliegenden Ende des zweiten Kommunikationsrohrs (3211) verbunden ist, wobei die zweite Schnittstelle mit dem zweiten Testende der zweiten Druckumwandlungsvorrichtung verbunden ist, wobei die dritte Schnittstelle mit der Mediumseinspritzvorrichtung verbunden ist.

7. Neuroelektrophysiologisches Überwachungsgerät (300) nach Anspruch 6, wobei das zweite Myoelektrizitätsmessglied an einem Außenumfang des zweiten Kommunikationsrohrs (3211) angeordnet ist, wobei das zweite Myoelektrizitätsmessglied aus einer Vielzahl von zweiten Elektromyografieelektroden besteht, wobei die Vielzahl von zweiten Elektromyografieelektroden in Längsrichtung des zweiten Kommunikationsrohrs (3211) oder in Umfangsrichtung des ersten Kommunikationsrohrs (3111) mit Abstand zueinander angeordnet sind.

8. Neuroelektrophysiologisches Überwachungsgerät (300) nach Anspruch 7, wobei eine Vielzahl von Skalierungsmarkierungen, die in Längsrichtung des zweiten Kommunikationsrohrs (3211) in Abständen angeordnet sind, an einer Außenwand des zweiten Kommunikationsrohrs (3211) vorgesehen sind.

## Revendications

1. Appareil de surveillance neuroélectrophysiologique (300), comprenant :
un dispositif de test urétral, le dispositif de test urétral étant pourvu d'un élément de mesure de la pression vésicale et d'un premier élément de mesure myoélectrique, l'élément de mesure de la pression vésicale étant conçu pour être placé dans la vessie d'un patient et adapté à mesurer la pression intravésicale du patient, le premier élément de mesure myoélectrique étant adapté à entrer en contact avec un sphincter urétral externe afin de mesurer l'activité électromyographique du sphincter urétral externe ;
un dispositif de test rectal, le dispositif de test rectal étant pourvu d'un élément de mesure de la pression rectale et d'un second élément de mesure myoélectrique, l'élément de mesure de la pression rectale étant conçu pour être placé dans le rectum du patient et adapté à mesurer la pression intrarectale du patient, le second élément de mesure myoélectrique étant adapté à entrer en contact avec un sphincter anal externe afin de mesurer l'activité électromyographique du sphincter anal externe ; et
un module de surveillance (33), le module de surveillance (33) étant électriquement connecté respectivement à l'élément de mesure de la pression vésicale, au premier élément de mesure myoélectrique, à l'élément de mesure de la pression rectale et au second élément de mesure myoélectrique afin de surveiller une activité électrophysiologique du nerf pudendal.

2. L'appareil de surveillance neuroélectrophysiologique (300) selon la revendication 1, comprenant en outre :
un premier tube de communication (3111), le premier tube de communication (3111) étant adapté à acheminer un milieu, une extrémité du premier tube de communication (3111) étant ouverte et placée dans la vessie du patient, l'extrémité opposée du premier tube de communication (3111) s'étendant à l'extérieur du corps du patient ; et
un premier dispositif de transduction de pression, le premier dispositif de transduction de pression ayant une première extrémité de test (3113) et une première extrémité de sortie de signal, la première extrémité de test (3113) communiquant avec le premier tube de communication (3111) et étant adaptée à mesurer une pression du milieu dans le premier tube de communication (3111), la première extrémité de sortie de signal étant connectée en communication au module de surveillance (33), le premier dispositif de transduction de pression étant adapté à convertir la pression du milieu dans le premier tube de communication (3111) en un signal de pression intravésicale et à transmettre le signal de pression intravésicale au module de surveillance (33) via la première extrémité de sortie de signal.

3. L'appareil de surveillance neuroélectrophysiologique (300) selon la revendication 2, dans lequel le premier élément de mesure myoélectrique est disposé à la périphérie externe du premier tube de communication (3111), dans lequel le premier élément de mesure myoélectrique est constitué d'une pluralité de premières électrodes d'électromyographie (3121), la pluralité de premières électrodes d'électromyographie (3121) étant agencée à intervalles réguliers dans une direction d'extension ou dans une direction périphérique du premier tube de communication (3111).

4. L'appareil de surveillance neuroélectrophysiologique (300) selon la revendication 2, comprenant en outre :
un dispositif d'ancrage du premier tube de communication (313), le dispositif d'ancrage du premier tube de communication (313) étant disposé à une extrémité du premier tube de communication (3111), le dispositif d'ancrage du premier tube de communication (313) étant conçu pour être sélectivement déformé afin d'être tendu dans la vessie du patient, une cavité extensible étant formée à l'intérieur du dispositif d'ancrage du premier tube de communication (313), la cavité extensible communiquant avec un tube de connexion à seringue (314) de telle sorte que le milieu est injecté dans la cavité extensible afin de contrôler la déformation du dispositif d'ancrage du premier tube de communication (313).

5. L'appareil de surveillance neuroélectrophysiologique (300) selon la revendication 2, dans lequel l'élément de mesure de la pression rectale comprend :
un second tube de communication (3211), le second tube de communication (3211) étant adapté à acheminer le milieu, une extrémité du second tube de communication (3211) étant conçue pour être placée dans le rectum du patient, l'extrémité opposée du second tube de communication (3211) s'étendant à l'extérieur du corps du patient ;
un ballon extensible intrarectal, le ballon extensible intrarectal étant disposé à une extrémité du second tube de communication (3211) et communiquant avec le second tube de communication (3211), une paroi périphérique externe du ballon extensible intrarectal étant conçue pour venir au contact de la paroi interne du rectum du patient ; et
un second dispositif de transduction de pression, le second dispositif de transduction de pression ayant une seconde extrémité de test et une seconde extrémité de sortie de signal, la seconde extrémité de test communiquant avec le second tube de communication (3211) et étant adaptée à mesurer une pression du milieu dans le second tube de communication (3211), la seconde extrémité de sortie de signal étant connectée en communication au module de surveillance, le second dispositif de transduction de pression étant adapté à convertir la pression du milieu en un signal de pression intrarectale et à transmettre le signal de pression intrarectale au module de surveillance via la seconde extrémité de sortie de signal.

6. L'appareil de surveillance neuroélectrophysiologique (300) selon la revendication 5, dans lequel l'extrémité opposée du second tube de communication (3211) est conçue pour être connectée à un dispositif d'injection de milieu, le dispositif d'injection de milieu injectant sélectivement le milieu dans le second tube de communication (3211),
dans lequel l'appareil de surveillance neuroélectrophysiologique (300) comprend en outre : un tube en T, le tube en T ayant une première interface, une deuxième interface et une troisième interface qui communiquent sélectivement entre elles, la première interface étant connectée à l'extrémité opposée du second tube de communication (3211), la deuxième interface étant connectée à la seconde extrémité de test du second dispositif de transduction de pression, la troisième interface étant connectée au dispositif d'injection de milieu.

7. L'appareil de surveillance neuroélectrophysiologique (300) selon la revendication 6, dans lequel le second élément de mesure myoélectrique est disposé à la périphérie externe du second tube de communication (3211), dans lequel le second élément de mesure myoélectrique est constitué d'une pluralité de secondes électrodes d'électromyographie, la pluralité de secondes électrodes d'électromyographie étant agencée à intervalles réguliers dans une direction d'extension du second tube de communication (3211) ou dans une direction périphérique du premier tube de communication (3111).

8. L'appareil de surveillance neuroélectrophysiologique (300) selon la revendication 7, dans lequel une pluralité de repères gradués agencés à intervalles dans la direction d'extension du second tube de communication (3211) sont disposés sur une paroi périphérique externe du second tube de communication (3211).
